(19) Europäisches Patentamt
European Patent Office
Office européen des brevets

(11) EP 1 751 122 B1

(12) FASCICULE DE BREVET EUROPEEN

(45) Date de publication et mention de la délivrance du brevet:
**17.03.2010 Bulletin 2010/11**

(21) Numéro de dépôt: **05769413.5**

(22) Date de dépôt: **04.05.2005**

(51) Int Cl.:
***C07D 253/065*** *(2006.01)*

(86) Numéro de dépôt international:
**PCT/FR2005/001129**

(87) Numéro de publication internationale:
**WO 2005/121108 (22.12.2005 Gazette 2005/51)**

# (54) DERIVES MONOMERIQUES DE 5,6-DIPHENYL-1,2,4-TRIAZINE ET LEURS UTILISATIONS

MONOMERE 5,6-DIPHENYL-1,2,4-TRIAZINDERIVATE UND DEREN VERWENDUNG

MONOMER 5,6-DIPHENYL-1,2,4-TRIAZINIC DERIVATIVES AND THE USE THEREOF

(84) Etats contractants désignés:
**AT BE BG CH CY CZ DE DK EE ES FI FR GB GR HU IE IS IT LI LT LU MC NL PL PT RO SE SI SK TR**

(30) Priorité: **05.05.2004 FR 0404810**

(43) Date de publication de la demande:
**14.02.2007 Bulletin 2007/07**

(73) Titulaire: **Pierre Fabre Dermo-Cosmétique**
**92100 Boulogne-Billancourt (FR)**

(72) Inventeurs:
- **REY, Jérôme**
**F-81100 Castres (FR)**
- **BORDAT, Pascal**
**F-31320 Mervilla (FR)**
- **TARROUX, Roger**
**F-31300 Toulouse (FR)**

(74) Mandataire: **Warcoin, Jacques et al**
**Cabinet Régimbeau**
**20, rue de Chazelles**
**75847 Paris Cedex 17 (FR)**

(56) Documents cités:
**FR-A- 2 477 873**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 751 122 B1

## Description

La présente invention se rapporte à des dérivés de 5,6-diphényl-1,2,4-triazine, et en particulier à leur utilisation comme filtres solaires sur la peau humaine et les cheveux ou comme agents protecteurs de la lumière dans l'industrie de matériaux synthétiques tels que les plastiques, les verres, les textiles. La présente invention a également pour objet les compositions cosmétiques contenant lesdits dérivés.

On rappellera brièvement que l'action du rayonnement solaire sur la peau dépend essentiellement de l'énergie des radiations qui atteignent les différentes couches cutanées. De façon générale, les radiations les plus énergétiques, i.e. possédant la longueur d'onde la plus faible (E=hc/λ), provoquent des érythèmes ou « coup de soleil », alors que les radiations moins énergétiques n'engendrent qu'un simple brunissage de la peau. On considère donc qu'un filtre solaire, destiné à entrer dans la composition des préparations cosmétologiques dites « anti-solaires » doit absorber au maximum les radiations de faible longueur d'onde, tout en restant transparent aux radiations de plus grande longueur d'onde.

Les photobiologistes ont pour habitude de diviser le spectre ultra-violet en trois portions appelées UV-A, UV-B et UV-C correspondant aux gammes de longueurs d'ondes décroissantes respectivement comprises entre 400 nm et 320 nm, entre 320 nm et 280 nm et entre 280 nm et 200 nm.

Les UV-B et UV-A permettent le brunissement de l'épiderme humain. Les UV-B provoquent des érythèmes et des brûlures cutanées qui peuvent nuire au développement du bronzage naturel. Pour ces raisons, ainsi que pour des raisons esthétiques, il existe une demande constante de moyens de contrôle de ce bronzage naturel en vue de contrôler la couleur de la peau. Il convient donc de filtrer ce rayonnement UV-B.

On sait également que les rayons UV-A, sont susceptibles d'induire une altération de la peau, notamment dans le cas des peaux sensibles ou des peaux continuellement exposées au rayonnement solaire. Les rayons UV-A provoquent en particulier une perte d'élasticité de la peau et l'apparition de rides conduisant à un vieillissement prématuré. Ils provoquent le déclenchement de réaction érythémateuse ou amplifient cette réaction chez certains sujets et peuvent même être à l'origine de réactions phototoxiques ou photoallergiques. Il est donc souhaitable de filtrer aussi le rayonnement UV-A.

Les UV-C, les plus fortement énergétiques créent des photokératites. L'ozone formé dans la stratosphère absorbe généralement une grande partie de ce rayonnement UV-C que l'on retrouve en revanche en quantité importante dans les rayonnements emis par les lampes artificielles, souvent responsables de graves accidents cutanés. Les UV-B qui pénètrent dans la couche de la peau et en particulier dans le corps muqueux de l'épiderme, provoquent des érythèmes solaires. Par suite, l'ensemble des radiations (UV-B + UV-C) constitue ce que l'on appelle la bande érythémale à l'égard de laquelle les filtres solaires doivent jouer le rôle d'écran. Les UV-A produisent la pigmentation directe de la peau (mélanogénèse), i.e. le brunissement de la peau.

Des composés dérivés de benzotriazoles et/ou benzothiazoles sont connus en tant que filtres UV, notamment dans le domaine cosmétique. La demande de brevet FR 2 803 194 décrit ainsi des dérivés de S-triazines portant des groupements phénylbenzothiazoles ou benzothiazoles utiles en tant que filtres UV sous forme particulaire. Ces composés couvrent le domaine de l'UV-A et de l'UV-B mais ils présentent l'inconvénient majeur d'absorber dans le visible (longueurs d'onde supérieure à 400 nm). Ces produits sont donc fortement colorés, ce qui limite leur utilisation dans les produits cosmétiques.

La présente invention propose de nouveaux dérivés de 5,6-diphényl-1,2,4-triazine capables d'absorber dans le domaine des UV-A et/ou des UV-B et/ou des UV-C, sans absorber dans le domaine du visible. Ces composés présentent donc l'avantage d'être peu colorés.

Ils présentent aussi l'avantage de pouvoir être spécifiques de l'un de ces domaines spectraux. Ceci est intéressant lorsque l'on souhaite filtrer un domaine UV précis (UV-A, UV-B ou UV-C), pour compléter par exemple l'efficacité spectrale d'un filtre UV qui présente une lacune sur ce domaine particulier.

Ces nouveaux dérivés offrent ainsi une gamme variée de filtres UV spécifiques et qui peuvent aussi présenter différents degrés d'absorbance. La combinaison de plusieurs de ces filtres sélectionnés en fonction de leur spécificité et leur degré d'absorbance, permet donc de préparer toute sorte de filtres UV agissant dans le spectre et avec l'absorbance désirés.

Ces nouveaux dérivés présentent aussi l'avantage d'être solubles dans divers excipients pharmaceutiquement acceptables, et de présenter une photostabilité meilleure que certains filtres commerciaux, ce qui les rend particulièrement utiles dans les produits cosmétiques, notamment les protections solaires.

La présente invention a pour objet l'utilisation comme filtres solaires actifs dans l'UV-A et/ou l'UV-B et/ou l'UV-C pour la peau humaine et/ou les cheveux, de composés 5,6-diphényl-1,2,4-triaziniques de formule générale (I) :

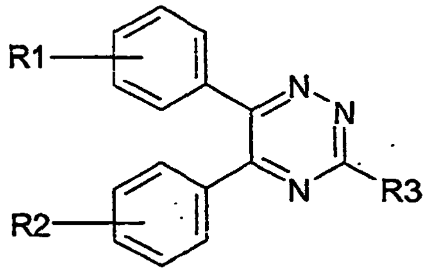

(I)

dans laquelle :

- les liaisons pénétrantes dans le cycle indiquent une position de substitution indifférente en ortho, méta ou para,
- $R_1$ et $R_2$, identiques ou différents, représentent un atome d'hydrogène, de fluor, de chlore ou de brome, un groupe alkyle linéaire ou ramifié en $C_1$ à $C_{12}$, hydroxy, alcoxy linéaire ou ramifié en $C_1$ à $C_{18}$, poly(éthoxy)-alcoxy avec un fragment alkyle en $C_1$ à $C_4$ et le nombre de motif éthoxy compris entre 1 et 4, amino, ou mono- ou di-alkylimino avec un fragment alkyle en $C_1$ à $C_4$,
- $R_3$ représente un atome de chlore, amino, phényle éventuellement substitué 1 à 3 fois par un radical hydroxy situé au moins en position para ou phényle éventuellement substitué 1 à 3 fois en position ortho, méta ou para, par un alcoxy en $C_1$ à $C_{12}$ ou un cyano et un groupe alkylimino avec un fragment alkyle en $C_1$ à $C_7$.

La présente invention a également pour objet l'utilisation des composés tels que précédemment définis comme agents protecteurs de la lumière actifs dans l'UV-A et/ou l'UV-B et/ou l'UV-C, utiles dans l'industrie des matériaux synthétiques, notamment comme agents protecteurs de la lumière entrant dans la composition de matières plastiques, de verre ou de matières textiles.

Ces composés objets de la présente invention peuvent ainsi être utilisés pour protéger les matériaux photosensibles.

L'agent de protection contre la lumière peut-être incorporé dans un substratum dans le but de protéger ce substratum contre l'attaque des rayons ultraviolets, pour empêcher la modification d'une ou de plusieurs propriétés physiques de ce substratum, comme par exemple la décoloration, une modification de la résistance au déchirage, une augmentation de la fragilité, etc.... et/ou des réactions chimiques provoquées par les rayons ultraviolets, par exemple dans un processus d'oxydation. Dans ce cas, on peut incorporer l'agent de protection avant et pendant la préparation du substratum, ou ultérieurement par un procédé convenable, par exemple un procédé de fixage analogue à une opération de teinture.

L'agent de protection contre la lumière peut aussi être incorporé dans un substratum pour protéger une ou plusieurs autres substances incorporées dans ledit substratum, par exemple des colorants, des agents auxiliaires, etc...

L'agent de protection contre la lumière peut aussi être incorporé dans une couche filtrante pouvant être solide (film, feuille) ou demi-solide (crème, huile, cire), que l'on applique sur un substratum, dans le but de protéger celui-ci des rayons ultraviolets.

Les composés de la présente invention conviennent non seulement comme agents de protection contre la lumière pour des matières incolores, mais également pour des matières pigmentées. Dans ce cas, la protection contre la lumière s'exerce aussi sur les colorants, permettant ainsi dans bien des cas une amélioration tout à fait notable de la stabilité à la lumière.

La présente invention a également pour objet les composés 5,6-diphényl-1,2,4-triaziniques de formule générale (Ia) :

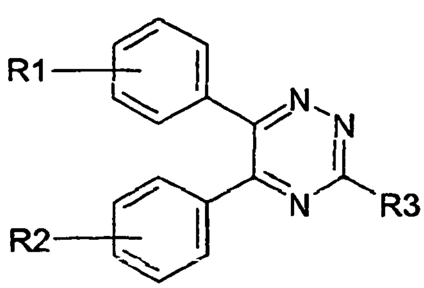

(Ia)

dans laquelle :

- $R_1$ et $R_2$ représentent un groupe $CH_3O$-, et
- $R_3$ représente :
  - un phényle substitué une ou plusieurs fois par un radical hydroxy situé au moins en position para,
  - un phényle substitué en position ortho, méta ou para, par un radical choisi parmi un alcoxy en $C_1$ ou $C_{12}$ ou un cyano,
  - ou un groupe alkylimino avec un fragment alkyle en $C_1$ à $C_7$.

Parmi les composés de formule générale (Ia), les composés suivants ont conduit à des résultats pratiques tout particulièrement intéressants :

- $R_1$ et $R_2$ représentent un groupe $CH_3O$- situé en position para, et
- $R_3$ représente un groupe phényle substitué par un radical hydroxy en position para, ou par un cyano ou un alcoxy en $C_1$ à $C_{12}$ en position ortho, méta ou para.

**[0021]** La présente invention a également pour objet les compositions cosmétiques anti-solaires contenant une quantité efficace d'au moins un composé de formule (Ia) en association avec un excipient cosmétiquement acceptable, de préférence entre 0,1 et 20 % en poids par rapport au poids total de la composition.

Les compositions cosmétiques anti-solaires selon l'invention peuvent contenir en outre un ou plusieurs filtres solaires complémentaires actifs dans l'UV-A et/ou dans l'UV-B et/ou dans l'UV-C (absorbeurs), hydrophiles ou lipophiles. Ces filtres complémentaires peuvent être notamment choisis parmi des dérivés cinnamiques, des dérivés dibenzoylméthane, des dérivés salicyliques, des dérivés du camphre et des dérivés de triazine autres que ceux précédemment cités dans la présente invention.

Les composés de formule générale (I) peuvent être préparés à partir de 1,2-dicétones de formule (II), par des méthodes conventionnelles connues de l'homme de l'art, comme celles décrites dans les exemples qui suivent.

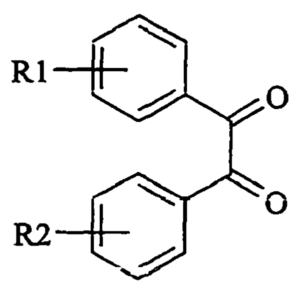

dicétone (II)

où $R_1$ et $R_2$ ont les mêmes significations que celles données précédemment.

Les dicétones de formule (II) sont disponibles dans le commerce (comme par exemple le benzil (diphényléthan-1,2-dione), 4,4'-diméthylbenzil, 4,4'-dibromobenzil, 4,4'-diflurorobenzil ou 4,4'-dichlorobenzil) ou peuvent être synthétisées par des méthodes conventionnelles bien connues de l'homme du métier. Par exemple, on peut utiliser la voie de synthèse suivante :

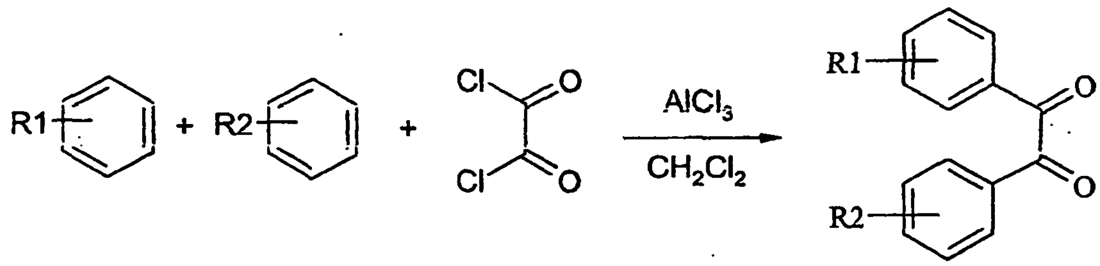


(II)

Les exemples qui suivent donnent d'autres exemples de synthèses des dicétones de formule (II).

On illustrera ci-après la présente invention en mentionnant quelques exemples non limitatifs de préparation de dérivés représentatifs répondant aux formules générales (I).

Les composés préparés sont rassemblés dans le tableau 1.

TABLEAU 1

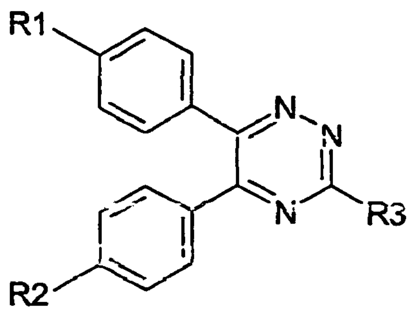

(I)

(où R1 et R2 sont en position para)

| **Référence** | **$R_1$, $R_2$** | **$R_3$** |
|---|---|---|
| Exemple comparatif JR18 | $-N(Et)_2$ | -OH |
| JR63 | -OMe | -Ph |
| JR65 | -OMe | -Ph(OH)p |
| JR67 | -H | -Ph(OH)o |
| JR68 | -H | -Ph(OH)p |
| JR70 | -OMe | -Ph(OMe)p |
| JR77 | -OMe | $-NH_2$ |
| JR89 | -OMe | -Ph(OH)o |
| JR98 | -OMe | -Cl |
| Exemple comparatif JR99 | -OMe | -OH |
| JR106 | -H | $-NH_2$ |
| JR107 | -OMe | -Ph(CN)p |
| JR113 | -OMe | -Ph(CN)m |
| JR114 | -OMe | -Ph(CN)o |
| JR115 | -OMe | $-Ph(OH)_3$ |

(suite)

| Référence | $R_1$, $R_2$ | $R_3$ |
|---|---|---|
| JR117 | -OMe | $-NCH(CH_2)_5CH_3$ |
| JR144 | -H | -Ph(CN)m |
| JR145 | -H | -Ph(CN)o |
| JR173 | -OMe | $-Ph(OC_{12}H_{25})p$ |

Les composés de formule (I) du tableau 1 peuvent être synthétisés de la façon suivante :

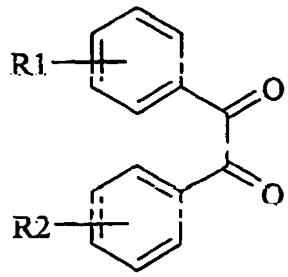

dicétone (II)

où R1, R2 = hydrogène, halogène
-OH,
alcoxy linéaire ou ramifié en $C_1$ à $C_{18}$ (tel que -OMe),
alkyle linéaire ou ramifié en $C_1$ à $C_{12}$,
mono ou dialkylamino avec un fragment alkyle en $C_1$ à $C_4$ (tel que $-N(Et)_2$).

dicétone (II)

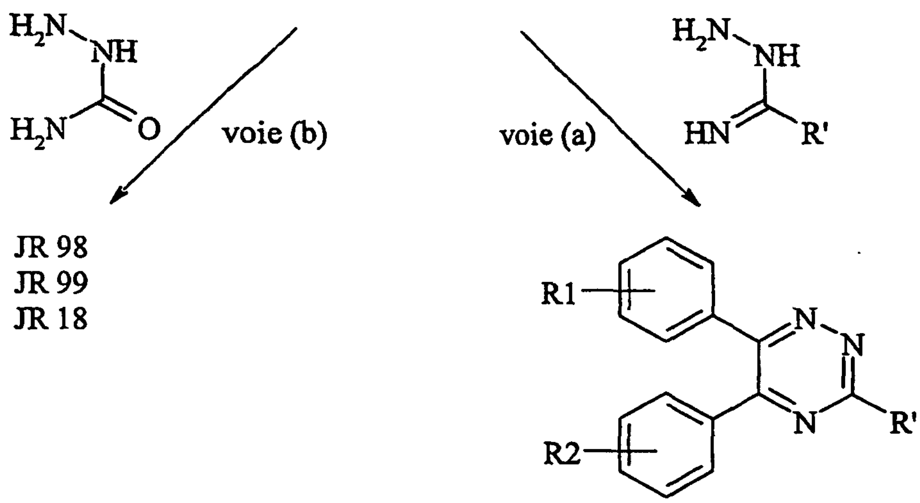


JR 98
JR 99
JR 18

où R' =
chlore
amino,
-OH,
phényle éventuellement substitué 1 à 3 fois par un radical -OH situé au moins en position para,
phényle éventuellement substitué 1 à 3 fois en position ortho, méta, ou para par un radical choisi parmi un alcoxy en $C_1$ à $C_{12}$ (tel que -OMe ou - $OC_{12}H_{25}$) ou un cyano,

alkylimino avec un fragment alkyle en $C_1$ à $C_7$ (tel que - $NCH(CH_2)_5CH_3$).

**FIGURES:**

DBM = Parsol 1789® (Laboratoires ROCHE)
MCX = Parsol MCX® (Laboratoires ROCHE)

La **figure 1** représente le spectre d'absorption dans l'UVA et l'UVB du Parsol 1789®.
La **figure 2** représente le spectre d'absorption dans l'UVA et l'UVB des composés JR18, JR70 et JR65 en comparaison avec celui du Parsol MCX®.
La **figure 3** représente le spectre d'absorption dans l'UVA et l'UVB des composés JR89, JR115, JR63, JR67, JR68 et JR77.
La **figure 4** représente le spectre d'absorption dans l'UVA et l'UVB des composés JR107, JR113 et JR114 en comparaison avec ceux du Tinosorb S® et du Tinosorb M®.

**Exemple 1 : Synthèse de la 1,2-bis(4-méthoxyphényl)-éthane-1,2-dione[1]**

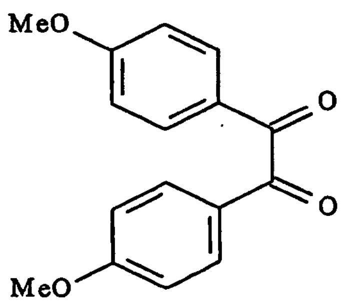

$C_{16}H_{14}O_4$ M=270,28 $g.mol^{-1}$

A un mélange d'anisole (10,8 g, 100 mmol) et de chlorure d'aluminium (33,33 g, 250 mmol) est ajouté lentement du chlorure d'oxalyle (4,71 ml, 55,2 mmol) à 0°C. Le mélange est agité à température ambiante pendant 4 heures. Après refroidissement, il est jeté dans de l'eau glacée et extrait au dichlorométhane. Les phases organiques rassemblées sont lavées par HCl 2N puis par de la saumure et séchées sur sulfate de magnésium. Après filtration et concentration sous pression réduite, le résidu est recristallisé dans de l'éthanol. Le précipité résultant est filtré, lavé plusieurs fois à l'éthanol et séché pour donner 9,80 g (66%) de produit pur sous la forme d'un solide jaune. $\delta_H$ (200 MHz, $CDCl_3$) 3,93 (s ; 6H), 6,99 (d; *J 7,8*; 4H), 7,99 (d; *J 7,8*; 4H).

**Exemple 2 : Synthèse de la 1.2-bis(4-diéthylaminophényl)-éthane-1.2-dione [2]**

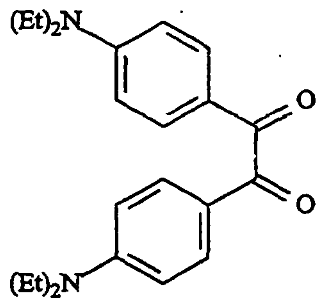

$C_{22}H_{28}N_2O_2$ M=352,48 g.mol$^{-1}$

A un mélange de N,N'-diéthylaniline (60 g, 382.1 mmol) et de chlorure d'aluminium (30.57 g, 229.3 mmol) est ajouté lentement du chlorure d'oxalyle (44.1 mL, 191.1 mmol) à 0°C. Le mélange est agité à température ambiante pendant 4 heures. Après refroidissement, il est jeté dans de l'eau glacée et extrait au dichlorométhane. Les phases organiques rassemblées sont lavées par HCl 2N puis par de la saumure et séchées sur sulfate de magnésium. Après filtration et concentration sous pression réduite, le résidu est recristallisé dans de l'éthanol. Le précipité résultant est filtré, lavé plusieurs fois à l'éthanol et séché pour donner 13,55 g (70%) de produit pur sous la forme d'un solide jaune. $\delta_H$ (200 MHz, $CDCl_3$), 1,25 (t, 12H), 3,45 (q, 8H), 6,65 (d; *J 7,8 ;* 4H), 7,87 (d; *J* 7,8; 4H).

**Exemple comparatif 3 : Synthèse de la 3-hydroxy-5,6-bis(4-diéthylaminophényl)-1,2,4-triazine (JR 18)**

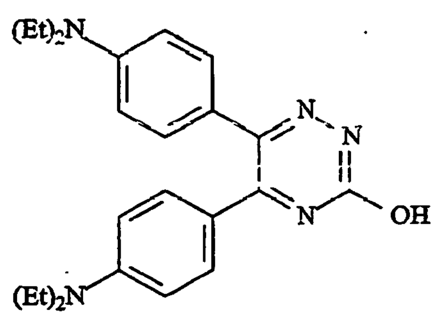

$C_{23}H_{29}N_5O$ M=391.52 g.mol$^{-1}$

A une solution de 1,2-bis(4-diéthylaminophényl)-éthane-1,2-dione préparée selon l'exemple 2 (5 g, 14,2 mmol) dans de l'acide acétique (20 mL) est ajouté le chlorydrate de semicarbazide (1,57 g, 14,2 mmol) et de l'acétate de sodium (1,41 g,, 17,3 mmol). Le milieu est chauffé à reflux pendant 12 heures. Après retour à température ambiante, le mélange réactionnel est versé dans de l'eau. Le solide brut est recueilli par filtration et lavé à l'eau. Le résidu est recristallisé dans de l'acide acétique. Après filtration et lavage à l'eau, 3 g (54%) d'un solide jaune sont obtenus après séchage. $\delta_H$ (200 MHz, $CDCl_3$), 1,18 (t ; 12H), 3,45 (q ; 8H), 6,65 (d; *J 7,8 ;* 4H), 7,87 (d; *J 7,8*; 4H), 10,4 (s ;1H)

**Exemple 4 : Synthèse de la 3-(2-hydroxyphényl)-5,6-bis(4-méthoxyphényl)-1,2,4-triazine (JR89)**

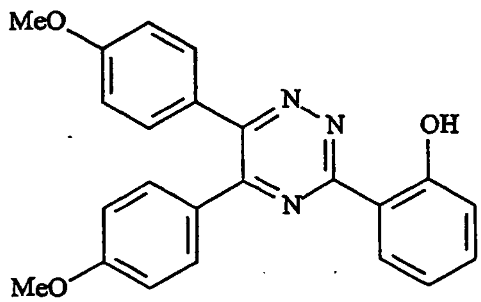

$C_{23}H_{19}N_3O_3$ M=385,42 g.mol$^{-1}$

A une solution de 1,2-bis(4-méthoxyphényl)-éthane-1,2-dione préparée selon l'exemple 1 (4 mg, 14,8 mmol) dans de l'acide acétique (20 mL) en présence d'acétate d'ammonium est ajouté du 2-hydroxyphénylhydrazide ( 2,23 g, 14,8 mmol). Le milieu est chauffé à reflux pendant 5 heures. Après retour à température ambiante, le précipité obtenu est filtré, lavé successivement à l'acide acétique puis à l'éthanol et séché pour donner un solide jaune. Le résidu est recristallisé dans un mélange diméthylformamide/ éthanol (95/5) pour donner 2,90 g (51 %) d'un solide jaune. $\delta_H$ (200 MHz, $CDCl_3$), 3,86 (s ; 6H), 7,02 (m; 4H), 7,58 (d; *J 7,8*; 2H), 7,71 (m ; 4H), 8,77 (d; *J 8,7*; 2H) , 12,3 (s ;1H), MS (Electrospray) *m/z* 386 ($MH^+$, 100%).

**Exemple 5 : Synthèse de la 3-(4-hydroxvphényl)-5,6-bis(4-méthoxyphényl)-1,2,4-triazine (JR65)**

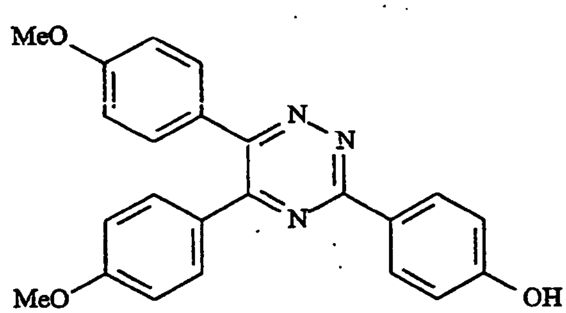

$C_{23}H_{19}N_3O_3$ M=385,42 g.mol$^{-1}$

A une solution de 1,2-bis(4-méthoxyphényl)-éthane-1,2-dione préparée selon l'exemple 1 (4 g, 14,8 mmol) dans de l'acide acétique (20 mL) en présence d'acétate d'ammonium est ajouté du 4-hydroxyphénylhydrazide (2,23 g, 14,8 mmol). Le milieu est chauffé à reflux pendant 6 heures. Après retour à température ambiante, le précipité obtenu est filtré, lavé successivement à l'acide acétique puis à l'éthanol et séché pour donner un solide jaune. Le résidu est purifié par chromatographie sur gel de silice (dichlorométhane/ éthanol : 95/ 5) pour donner 2,56 g (45 %) d'un solide jaune. $\delta_H$ (200 MHz, $CDCl_3$), 3,86 (s ; 6H), 7,02 (m; 4H), 7,58 (d; *J* 7,8; 2H), 7,71 (m; 4H) ,8,77 (d; *J 8,7*; 2H), 11,79 (s ; 1H), MS (Electrospray) *m/z* 386 ($MH^+$; 100%).

**Exemple 6 : Synthèse de la 3-(2-hydroxyphényl)-5,6-diphényl-1,2,4-triazine (JR67)[3)]**

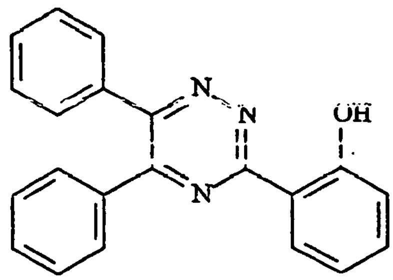

$C_{21}H_{15}N_3O$ M=325,37 $g.mol^{-1}$

A une solution de benzil (4 mg, 19 mmol) dans de l'acide acétique (20 mL) en présence d'acétate d'ammonium est ajouté du 2-hydroxyphénylhydrazide (3,17 g, 20,9 mmol). Le milieu est chauffé à reflux pendant 4h30 heures. Après retour à température ambiante, le précipité obtenu est filtré, lavé successivement à l'acide acétique puis à l'éthanol et séché pour donner un solide jaune. Le résidu est recristallisé dans un mélange diméthylformamide/ éthanol (90/10) pour donner 3,15 g (51%) d'un solide jaune. $\delta_H$ (200 MHz, $CDCl_3$), 7,02 (m; 5H), 7,58 (d; *J* 7,8; 2H), 7,71 (m; 4H), 8,77 (d; *J 8,7*; 2H), 12,6 (s ;1H), MS (Electrospray) *m/z* 359 ($MH^+$; 100%), 673 ($2M+Na^+$; 21%).

**Exemple 7 : Synthèse de la 3-(4-hydroxyphényl)-5,6-diphényl-1,2,4-triazine (JR68)**

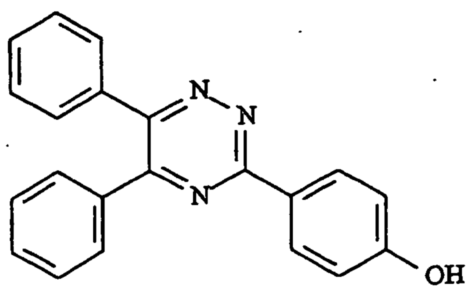

$C_{21}H_{15}N_3O$ M=325,37 $g.mol^{-1}$

A une solution de benzil (4 g, 19 mmol) dans de l'acide acétique (20 mL) en présence d'acétate d'ammonium est ajouté du 4-hydroxyphénylhydrazide (3,17 mg, 20,9 mmol). Le milieu est chauffé à reflux pendant 3 heures. Après retour à température ambiante, le précipité obtenu est filtré, lavé successivement à l'acide acétique puis à l'éthanol et séché pour donner un solide jaune. Le résidu est purifié par chromatographie sur gel de silice (dichlorométhane/ éthanol : 94/ 6) pour donner 3,46 g (56%) d'un solide jaune. $\delta_H$ (200 MHz, $CDCl_3$), 7,02 (m; 5H), 7,58 (d; *J 7,8*; 2H), 7,71 (m; 4H), 8,77 (d; *J 8,7*; 2H), 11,79 (s ;1H), MS (Electrospray) *m/z* 326 ($MH^+$, 100%).

**Exemple 8: Synthèse de la 3-(4-méthoxyphényl)-5.6-bis(4-méthoxphényl)-1,2,4-triazine- (JR70)**

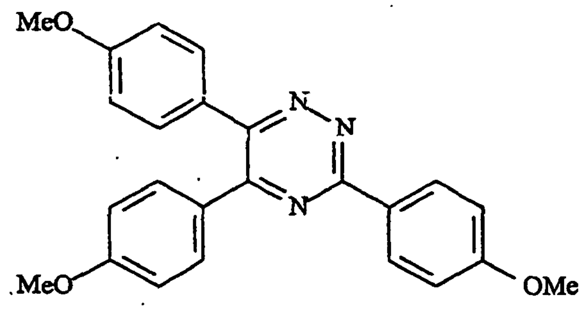

$C_{24}H_{21}N_3O_3$ M= 399,45 g.mol$^{-1}$

A une solution de 1,2-bis(4-méthoxyphényl)-éthane-1,2-dione préparée selon l'exemple 1 (4 g, 14,8 mmol) dans de l'acide acétique (20 mL) en présence d'acétate d'ammonium est ajouté du 4-méthoxyphénylhydrazide (2,23 g, 14,8 mmol). Le milieu est chauffé à reflux pendant 5 heures. Après retour à température ambiante, le précipité obtenu est filtré, lavé successivement à l'acide acétique puis à l'éthanol et séché pour donner un solide jaune. Le résidu est purifié par chromatographie sur gel de silice (dichlorométhane/ éthanol : 95/ 5) pour donner 2,42 g (41 %) d'un solide jaune. (200 MHz, $CDCl_3$), 3,86 (s; 6H),3,90 (s,3 H), 7,02 (m; 4H), 7,58(d; *J* 7,8; 2H), 7,71 (m; 4H), 8,63 (d; *J 8,7*; 2H), MS (Electrospray) *m/z* 460 ($MH^+$, 100%).

**Exemple 9 : Synthèse de la 3-(3,4,5-hydroxyphényl)-5,6-bis(4-méthoxyphényl)-1,2,4-triazine (JR115)**

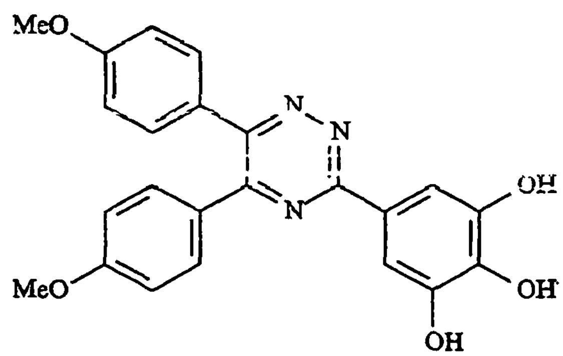

$C_{23}H_{19}N_3O_5$ M= 417,42 g.mol$^{-1}$

A une solution de 1,2-bis(4-méthoxyphényl)-éthane-1,2-dione préparée selon l'exemple 1 (3 g, 11 mmol) dans de l'acide acétique (20 mL) en présence d'acétate d'ammonium est ajouté du 3,4,5-trihydroxyphénylhydrazide ( 2,5g, 11 mmol). Le milieu est chauffé à reflux pendant 5h30 min. Après retour à température ambiante, le précipité obtenu est filtré, lavé successivement à l'acide acétique puis à l'éthanol et séché pour donner un solide jaune. Le résidu est purifié par chromatographie sur gel de silice (dichlorométhane/ éthanol 90/ 10) pour donner 200 mg (5%) d'un solide jaune. $\delta_H$ (200 MHz, $CDCl_3$, 3,86 (s, 6H), 7,02 (m; 4H), 7,58 (d; *J 7,8*; 2H), 7,71 (d; *J 8,7*; 2H), 8,70 (s; 2H), 7,71(m; 4H), 8,77(d; *J 8,7*; 2H), 11,79 (s ;1H), 11,90 (2H ; s), MS (Electrospray) *m/z* 418 ($MH^+$, 100%).

**Exemple 10 : Synthèse de la 3-(4-dodécyloxyphényl)-5,6-bis(4-méthoxyphényl)-1,2,4-triazine (JR173)**

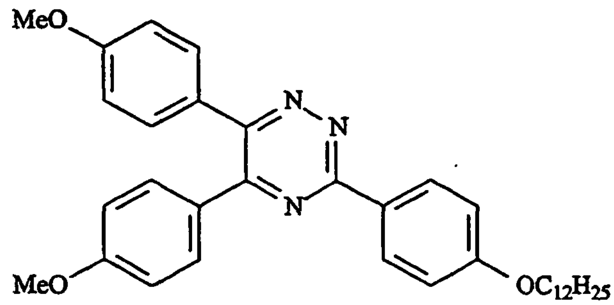

$C_{35}H_{43}N_3O_3$ M= 553,74 g.mol$^{-1}$

A une solution d'hydroxyde de sodium (872 mg, 21,8 mmol) dans l'éthanol (50 mL) est ajouté JR65 préparé selon l'exemple 5 (7 g, 18,1 mmol). Le milieu est agité une demi-heure. A la solution est ajoutée le 1-bromododécane (9,473 g, 38 mmol). Le tout est porté à reflux dans l'éthanol pendant 12 heures. Après retour à température ambiante, le précipité obtenu est filtré et recristallisé par deux fois dans du cyclohexane pour donner 5,2g ( 52%) d'un solide jaune. $\delta_H$ (200 MHz, $CDCl_3$), 0,92 (m; 3H), 1,20-1,40 (m; 19 H), 1,50-1,87 (m ; 2H), 3,91 (s ; 6H), 4,00 (t; *J 7,5*, 2H) 6,95 (m; 4H), 7,65 (d; *J 7,8*; 2H), 7,82 (m; 4H),. 8,63 (d; *J 8,7*; 2H), MS (Electrospray) *m/z* 554 ($MH^+$, 100%).

**Exemple 11 : Synthèse de la 3-amino-5,6-bis(4-méthoxyphényl)-1,2,4-triazine (JR77)**

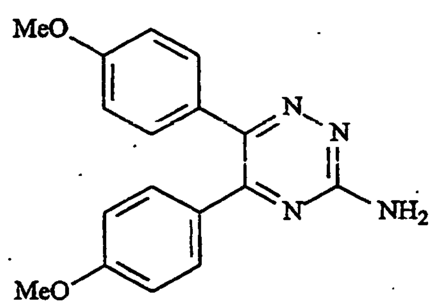

$C_{17}H_{16}N_4O_2$ M=308,34 g.mol$^{-1}$

A une solution de 1,2-bis(4-méthoxyphényl)-éthane-1,2-dione préparée selon l'exemple 1 (5 g, 18,5 mmol) dans de l'éthanol (50 mL) est ajouté l'aminoguanidine (2,450 g, 2,21 mmol). Le milieu est chauffé à reflux pendant 12 heures. Après retour à température ambiante, le précipité obtenu est filtré, lavé à l'éthanol et séché pour donner un solide jaune. Le résidu est purifié par chromatographie sur gel de silice (dichlorométhane/ triéthylamine 0,5%) pour donner 3,7g (65%) d'un solide jaune. $\delta_H$ (200 MHz, $CDCl_3$), 3,88 (s ; 6H), 5,51 (s ; 2H), 6,92 (m; 4H), 7,41 (m; 4H).

**Exemple 12: [5.6-bis-(4-méthoxyphényl)-[1,2,4]-triazin-3-yl]heptylidine-amine (JR117)**

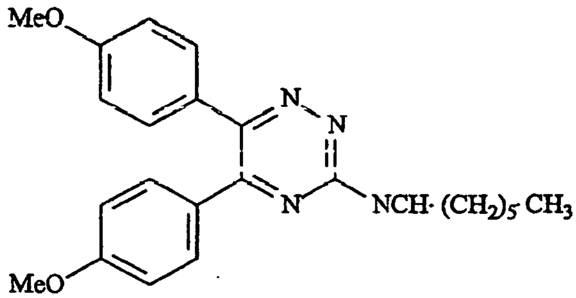

$C_{24}H_{28}N_4O_2$ M=404,50 g.mol$^{-1}$

JR77 préparée selon l'exemple 11 (2 g, 6,5 mmol) et l'heptaldéhyde ( 0,907 ml, 6,5 mmol) sont mis en solution dans de l'acide acétique glacial et chauffés à reflux à pendant 1h30 min. Après refroidissement, le brut réactionnel est précipité sur de la glace pilée. Le solide obtenu est remis en solution dans du dichlorométhane et filtré. Le solvant est évaporé à sec, 2 g d'une huile jaune sont obtenus. $\delta_H$ (200 MHz, $CDCl_3$), 0,92 (m; 3H), 1,29 (m; 6 H), 1,59 (m ; 2H), 2,34 (t; *J 7,5*, 2H) 3,86 (s ; 6H), 6,77 (d ; 2H) 6,89 (d ; 2H), 7,44 (m; 4H), MS (Electrospray) *m/z* 405 ($MH^+$, 100%).

**Exemple 13 : Synthèse de la 2-cyanophénylcarboxamidehydrazone**

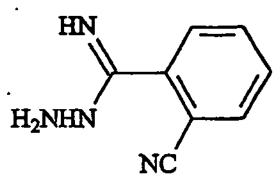

$C_8H_8N_4$ M=160,18 g.mol$^{-1}$

Un mélange de 1,2-dicyanobenzène (10 g, 78,1 mmol) et d'hydrazine monohydrate (50 ml) est mis sous atmosphère d'azote. Le mélange est agité à température ambiante pendant 12 heures. Le précipité jaune obtenu est alors filtré et mis à recristalliser dans de l'éthanol pour donner 5 g (40%) d'un solide de couleur jaune. $\delta_H$ (200 MHz, acétone-*d*), 3,53 (m ; 4H), 7,72 (m; 1H), 8,05 (m; 2H), 8,58 (s ; 1H).

**Exemple 14 : Synthèse de la 3-cyanophénylcarboxamidehydrazone**

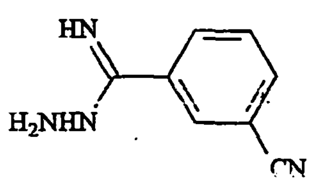

$C_8H_8N_4$ M=160,18 g.mol$^{-1}$

Un mélange de 1,3-dicyanobenzène (10 g, 78,1 mmol) et d'hydrazine monohydrate (50 ml) est mis sous atmosphère d'azote. Le mélange est agité à température ambiante pendant 12 heures. Le précipité jaune obtenu est alors filtré et mis à recristalliser dans de l'éthanol pour donner 5,62 g (45%) d'un solide de couleur jaune. $\delta_H$ (200 MHz,

$CDCl_3$), 3,53 (m ; 4H), 7,50 (t; *J* 7,3; 1H), 7,67 (d ; *J* 7,3; 2H), 7,95 (m ; 1H).

**Exemple 15 : Synthèse de la 4-cyanophénylamidrazone**

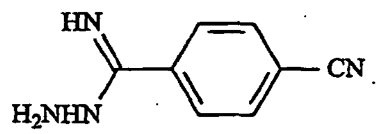

$C_8H_8N_4$ $M=160,18\ g.mol^{-1}$

Un mélange de 1,4-dicyanobenzène (10 g, 78,1 mmol) et d'hydrazine monohydrate (50 ml) est mis sous atmosphère d'azote. Le mélange est agité à température ambiante pendant 12 heures. Le précipité jaune obtenu est alors filtré et mis à recristalliser dans de l'éthanol. Pour donner 5,75 g (46%) d'un solide de couleur jaune. $\delta_H$ (200 MHz, $CDCl_3$) 3,51 (m ; 4H) 7,70 (d ; *J* 7,3 ; 2H), 7,95 (*J* ; 7,3 ; 2H).

**Exemple 16 : Synthèse de la 3-(3-cyanophényl)-5,6-diphényl-1,2,4-triazine (JR144)**

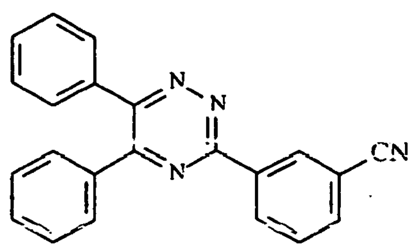

$C_{22}H_{14}N_4$ $M= 334,00\ g.mol^{-1}$

A une solution de benzil (2,14g, 10,2 mmol) dans du diméthylsulfoxyde (DMSO) (100ml) est ajoutée le 3-cyanophénylcarboxamidehydrazone préparé selon l'exemple 14 (1 g, 6,25 mmol). Le milieu est chauffé à reflux pendant 15h30 min à 130°C. Après retour à température ambiante, le brut réactionnel est précipité dans l'eau, le précipité obtenu est filtré, puis séché. Le résidu est purifié par chromatographie sur gel de silice (dichlorométhane) pour donner 1,2 g ( 57%) d'un solide jaune. $\delta_H$ (200 MHz, $CDCl_3$), 7,35 (m; 5H), 7,65 (d; *J 7.8*; 2H), 7,82 (d; *J 8,7*; 2H), 8,63 (s; 4H), MS (Electrospray) *m/z* 335 ($MH^+$, 100%).

**Exemple 17: Synthèse de la 3-(2-cyanophényl) -5,6-diphénvl-1,2,4-triazine (JR145)**

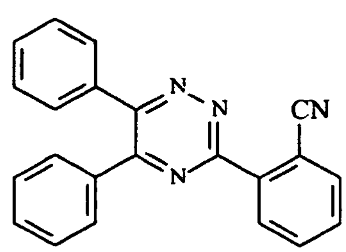

$C_{22}H_{14}N_4$ $M= 334,00\ g.mol^{-1}$

A une solution de benzil (2,142 g, 10,2 mmol) dans du diméthylsulfoxyde (DMSO) (100ml) est ajoutée le 2-cyanophénylcarboxamidehydrazone préparé selon l'exemple 13 (1 g, 6,25 mmol). Le milieu est chauffé à reflux pendant 15h30 min à 130°C. Après retour à température ambiante, le brut réactionnel est précipité dans l'eau, le précipité obtenu est filtré, puis séché. Le résidu est purifié par chromatographie sur gel de silice (dichlorométhane) pour donner 1,1 g (55%) d'un solide jaune. $\delta_H$ (200 MHz, $CDCl_3$), 7,34 (m; 5H), 7,65 (m; 3H), 7,82 (s; 1H), 8,63 (s; 4H), MS (Electrospray) *m/z* 335 ($MH^+$, 100%).

**Exemple 18: Synthèse de la 3-(2-cyanophényl)-5,6-bis(4-méthoxyuhényl)-1,2,4-triazine (JR114)**

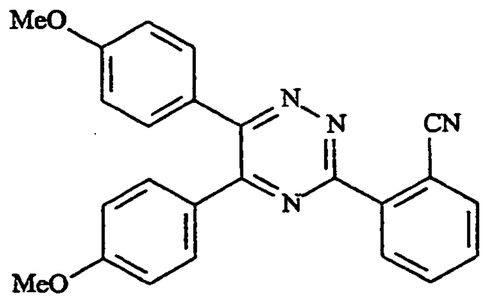

$C_{24}H_{18}N_4O_2$ M= 394,43 $g.mol^{-1}$

A une solution de 1,2-bis(4-méthoxyphényl)-éthane-1,2-dione préparée selon l'exemple 1 (2,75 g, 10,2 mmol) dans l'éthanol (20 mL) est ajoutée le 2-cyanophénylcarboxamidehydrazone préparé selon l'exemple 13 (1g, 6,25 mmol). Le milieu est chauffé à reflux pendant 9h30min. Après retour à température ambiante, le solvant est évaporé et le solide est purifié par chromatographie sur gel de silice (dichlorométhane/ éthanol : 95/5). Nous obtenons 1,653g (40%) d'un produit jaune orange. $\delta_H$ (200 MHz, $CDCl_3$), 3,86 (s ; 6H), 6,88 (m; 4H), 7,58 (d; *J 7,8*; 2H), 7,82 (m; 4H), 8,77 (d; *J 8,7*; 2H), MS (Electrospray) *m/z* 395 ($MH^+$, 100%).

**Exemple 19 : Synthèse de la 3-(3-cyanophényl)-5,6-bis(4-méthoxyphényl)-1,2,4-triazine (JR113)**

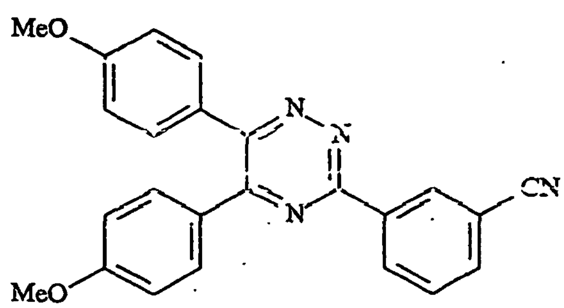

$C_{24}H_{18}N_4O_2$ M=394,43 $g.mol^{-1}$

A une solution de 1,2-bis(4-mémoxyphényl)-éthane-1,2-dione préparée selon l'exemple 1 (4,2 g, 15,6 mmol) dans l'éthanol (20 mL) est ajoutée le 3-cyanophénylcarboxamidehydrazone préparé selon l'exemple 14 (1,5 g, 9,37 mmol). Le milieu est chauffé à reflux pendant 10 heures. Après retour à température ambiante, le solvant est évaporé et le solide est purifié par chromatographie sur gel de silice (dichlorométhane/ éthanol: 96/4). Après évaporation du

solvant, nous obtenons 963 mg (37%) d'un solide jaune. $\delta_H$ (200 MHz, $CDCl_3$), 3,91 (s ; 6H), 6,95 (m; 4H), 7,65 (m; 3H), 7,82 (m; 4H), 8,63 (m; 2H), MS (Electrospray) *m/z* 395 ($MH^+$, 100%).

**Exemple 20 : Synthèse de la 3-(4-cyanophényl)-5,6-bis(4-méthoxyphényl)-1,2,4-triazine (JR107)**

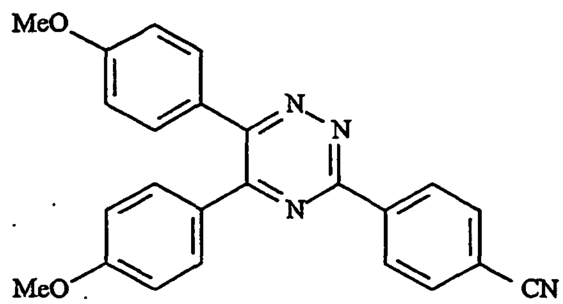

$C_{24}H_{18}N_4O_2$ M=394,43 $g.mol^{-1}$

A une solution de 1,2-bis(4-méthoxyphényl)-éthane-1,2-dione préparée selon l'exemple 1 (2,81 g, 10,41 mmol) dans l'éthanol (20 mL) est ajoutée le 4-cyanophénylcarboxamidehydrazone préparé selon l'exemple 15 (1 g, 6,25 mmol). Le milieu est chauffé à reflux pendant 12 heures. Après retour à température ambiante, le solvant est évaporé et le solide est purifié par chromatographie sur gel de silice (dichlorométhane/ éthanol : 96/4). Après évaporation du solvant, nous obtenons 1,92 g (78%) d'un solide jaune. $\delta_H$ (200 MHz, $CDCl_3$), 3,91 (s ; 6H), 6,95 (m; 4H), 7,65 (d; *J 7,8*; 2H), 7,82 (m; 4H), 8,63 (d; *J 8,7*; 2H) MS (Electrospray) *m/z* 395 ($MH^+$, 100%).

**Exemple comparatif 21 : Synthèse de la 3-hydroxy-5,6-bis(4-méthoxyphényl)-1,2,4-triazine (JR99)(4)**

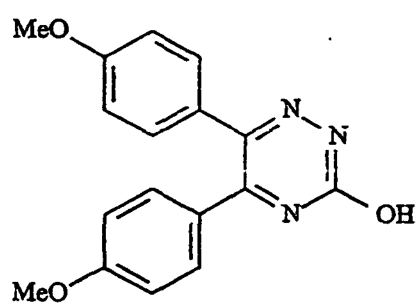

$C_{17}H_{15}N_3O_3$ M=309,32 $g.mol^{-1}$

A une solution de 1,2-bis(4-méthoxyphényl)-éthane-1,2-dione préparée selon l'exemple 1 (5,4 g, 20 mmol) dans de l'acide acétique (25 mL) est ajouté le chlorydrate de semicarbazide (2,22 g, 20 mmol) et de l'acétate de sodium (1.80 g, 22 mmol). Le milieu est chauffé à reflux pendant 12 heures. Après retour à température ambiante, le mélange réactionnel est versé dans de l'eau. Le solide brut est recueilli par filtration et lavé à l'eau. Le résidu est recristallisé dans de l'acide acétique. Après filtration et lavage à l'eau, 5,9 g (95%) d'un solide jaune est obtenu après séchage. $\delta_H$ (200 MHz, $CDCl_3$), 3,86 (s ; 6H), 6,94 (m; 4H), 7,67 (s; 4H).

**Exemple 22: Synthèse de la 3-chloro-5,6-bis(4-méthoxyphényl)-1,2,4-triazine (JR98)[4]**

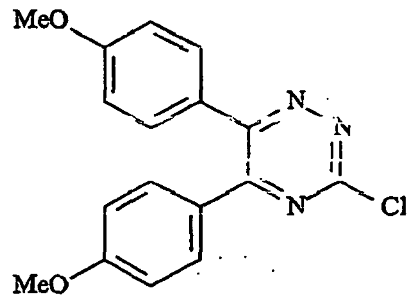

$C_{17}H_{14}ClN_3O_2$ M=327,77 $g.mol^{-1}$

JR99 préparé selon l'exemple 21 (4,57 g, 14,8 mmol) en solution dans 23 ml d'oxychlorure de phosphore est porté à reflux pendant 1h 50 min. Le mélange refroidi est versé sur de la glace pilée et extrait avec de l'éther diéthylique. L'extrait est lavé successivement avec une solution à 2% d'hydroxyde de sodium et de l'eau jusqu'à ce que les produits de lavage soient neutres. L'extrait éthéré est séché sur sulfate de sodium anhydre et évaporé. Le résidu est repris dans l'éther et filtré. Le filtrat est évaporé et donne 3,5 g (72%) d'un solide jaune marron. $\delta_H$ (200 MHz, $CDCl_3$), 3,86 (s ; 6H), 6,94 (m; 4H), 7,67 (s; 4H).

**Références bibliographiques des synthèses :**

(1) G. Pitet, H. Cousse, G. Mouzin, Boll. Chim. Farm., 1980, 119, 469
(2) C. Tuzin, M. Ogliaruso, E. I. Becker, Org. Syn.,1961, 41, 3
(3) E. C. Taylor, L. G. French, J. Org. Chem, 1989, 54, 1245
(4) US Patent 1976 n° 3948894.


On trouvera ci-après les études physico-chimiques réalisées sur les composés objets de la présente invention, en comparaison avec les filtres commerciaux suivants :

PARSOL 1789® :

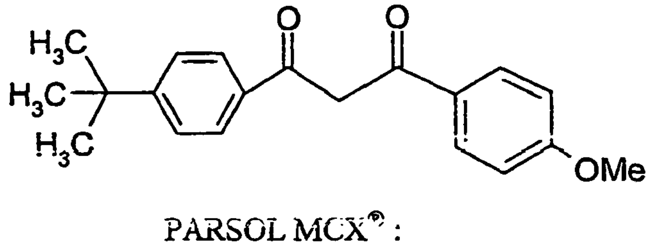

PARSOL MCX® :

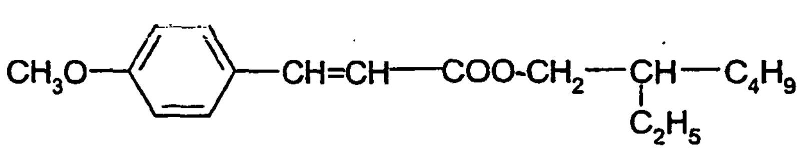

TINOSORB S® :

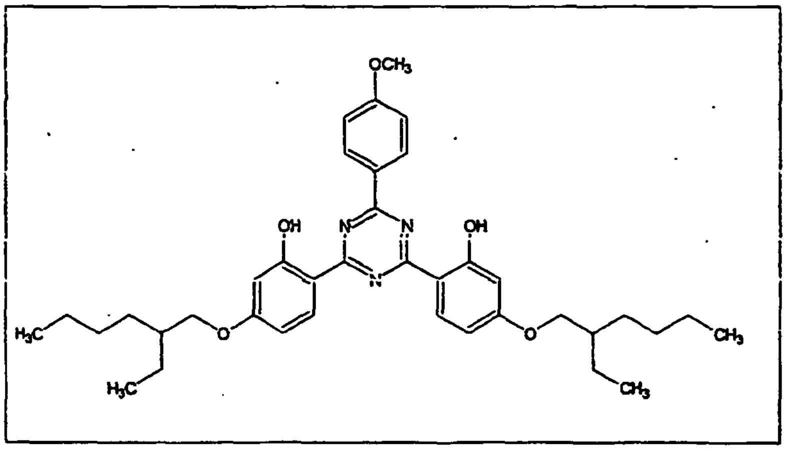

TINOSORB M® :

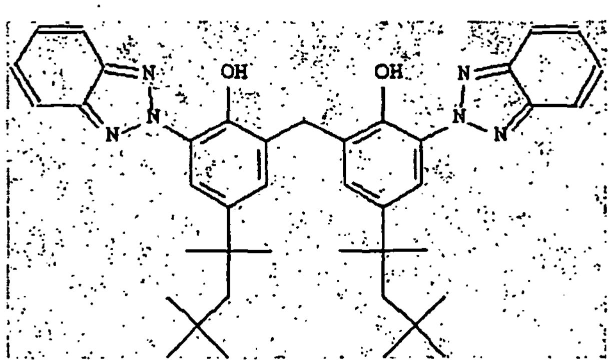

**Exemple 23 : Caractéristiques spectrales des produits : coefficient d'extinction molaire et l'absorbance spécifique**

Le calcul du coefficient d'extinction molaire (ε) se fait à partir de la loi de Beer-Lambert :

$$Log_{10}\left(\frac{I_0}{I}\right) = \varepsilon.l.c = A$$

Ou:

A= absorbance
$I_0$=intensité de la lumière incidente
I= intensité de la lumière transmise
ε= coefficient d'extinction molaire ( ou absorbance molaire ) en $M^{-1}$. $cm^{-1}$
1= longueur de la cuve en cm
c= concentration en mol. $L^{-1}$

Le coefficient d'extinction molaire peut-être exprimé par rapport à une masse donnée de produit. Il permet alors de pouvoir comparer les coefficients d'extinction entre eux pour une même quantité de produit donné. Cette quantité

est de 1% en poids. Le coefficient d'extinction molaire devient alors l'*absorbance spécifique* ( $A_{1cm}^{1\%}$ ).

Il s'exprime comme il suit :

$$A_{1cm}^{1\%} = \varepsilon.\frac{10}{M}$$

Où:

$A_{1cm}^{1\%}$ = l'absorbance spécifique

ε=coefficient d'extinction molaire

M= masse molaire

Les caractéristiques spectrales des composés en comparaison avec des filtres commerciaux à une concentration de 10 $\mu$g.ml$^{-1}$ sont regroupées dans les tableaux 2-1 et 2-2.

**Mode opératoire** : Les produits sont dissous dans de l'acétate d'éthyle à une concentration de 10 $\mu$g.ml$^{-1}$. Les spectres sont effectués à l'aide d'un spectrophotomètre (Varian CARY 50 Scan) à double faisceau entre 290 nm et 400 nm.

**TABLEAU 2-1**

| | Coefficient d'extinction molaire maximal | | | | Absorbance spécifique maximale | | | |
|---|---|---|---|---|---|---|---|---|
| **Molécules** | **UVC** | **UVB** | **UVA** | **Visible** | **UVC** | **UVB** | **UVA** | **Visible** |
| Parsol 1789® | 8633 à 275 | | 34100 à 360 | | 278 à 275 | | 1100 à 360 | |
| Parsol MCX® | | 24600 à 310 | | | | 848 à 310 | | |
| Tinosorb S® | | 47150 à 310 | 49580 à 345 | | | 751 à 310 | 789 à 345 | |
| Tinosorb M® | | 36600 à 305 | 36400 à 345 | | | 556 à 305 | 552 à 345 | |

**TABLEAU 2-2**

| Composés de formule (I) | | | | | | | | |
|---|---|---|---|---|---|---|---|---|
| | Coefficient d'extinction molaire maximal | | | | Absorbance spécifique maximale | | | |
| **Molécule** | **UVC** | **UVB** | **UVA** | **Visible** | **UVC** | **UVB** | **UVA** | **Visible** |
| **JR89** | 19300 à 280 | | 20300 à 335 | | 502 à 280 | | 526 à 335 | |
| **JR115** | | 34300 à 310 | | | | 747 à 310 | | |
| **JR18** | | 23100 à 290 | | 19000 à 435 | | 591 à 290 | | 483 à 435 |
| **JR63** | | 22736 à 295 | | | | 616 à 295 | | |
| **JR65** | | 31100 à 315 | | | | 809 à 315 | | |
| **JR67** | 26300 à 280 | | 16300 à 330 | | 811 à 280 | | 502 à 330 | |

(suite)

| Composés de formule (I) | | | | | | | | |
|---|---|---|---|---|---|---|---|---|
| | Coefficient d'extinction molaire maximal | | | | Absorbance spécifique maximale | | | |
| Molécule | UVC | UVB | UVA | Visible | UVC | UVB | UVA | Visible |
| JR68 | 22800 à 280 | | 13800 à 330 | | 702 à 280 | | 424 à 330 | |
| JR70 | | 29200 à 310 | | | | 982 à 310 | | |
| JR77 | 19300 à 280 | | 7600 à 360 | | 631 à 280 | | 249 à 360 | |
| JR107 | 25800 à 270 | 27100 à 300 | 19600 à 350 | | 655 à 270 | 688 à 300 | 498 à 350 | |
| JR113 | | 28800 à 295 | 22469 à 340 | | | 721 à 295 | 570 à 340 | |
| JR114 | | | 26000 à 325 | | | | 661 à 352 | |
| JR173 | | 48600 à 305 | | | | 878 à 305 | | |
| * comparatif | | | | | | | | |

**Exemple 24 :**

Les produits testés sont classés suivant la répartition spectrale dans l'UVA et l'UVB dans un domaine allant de 290 à 400nm.

Il est possible de les différencier en fonction de leur répartition spectrale : Produits à spectre étroit :

- Les produits absorbant dans la zone comprise entre 280 nm et 320 nm (UVB)
- Les produits absorbant dans la zone comprise entre 320 nm et 400 nm (UVA)

Produits à large spectre :

- Les produits absorbant dans l'UVB (280 nm - 320 nm) et l'UVA-II (380 nm - 360 nm)
- Les produits couvrant l'UVB et l'UVA (280 nm - 400 nm).

**Exemple 24-1 : Répartition spectrale des composés de formule (I)**

Parsol 1789® n'absorbe que dans l'UVA (voir figure 1).

JR18, JR70, JR65, JR173 absorbent dans l'UVB (voir figure 2).

JR89, JR115, JR63, JR68 et JR77 absorbent dans l'UVB (280 nm - 320 nm) et l'UVA-II (280 nm - 340 nm) (voir figure 3).

JR107, JR113, JR114, Tinosorb S® et Tinosorb M® absorbent dans l'UVB et l'UVA (voir figure 4).

**Exemple 24-2 :**

Le tableau 3 résume les répartitions spectrales des composés testés.

**TABLEAU 3**

| Molécules | UVB 280-320 nm | UVA-II 320-340 nm | UVA-I 340-400 nm | Pics d'absorption ( nm) |
|---|---|---|---|---|
| Parsol 1789® | | | | 360 |
| WP76 | | | | 290 |
| Parsol® | | | | 310 |
| JR18 * | | | | 290 |
| JR65 | | | | 315 |
| JR70 | | | | 310 |
| JR173 | | | | 305 |
| JR89 | | | | 335 |
| JR115 | | | | 310 |
| JR63 | | | | 295 |
| JR68 | | | | 280 et 330 |
| JR70 | | | | |
| JR77 | | | | 310 |
| JR107 | | | | 270-300-350 |
| JR113 | | | | 295 et 340 |
| JR114 | | | | 325 |
| Tinosorb S® | | | | 310 et 345 |
| Tinosorb M® | | | | 305 et 345 |

* comparatif

- Très forte absorption
- Forte absorption
- Absorption moyenne
- Absorption faible
- Absorption nulle

**Exemple 25 : Evaluation du facteur de protection solaire (FPS) *in vitro* dans un solvant chimique ou dans une formule primaire**

Les méthodes *in vitro* de détermination de l'efficacité protectrice des produits solaires consistent à mesurer par spectrophotométrie de transmission le spectre d'absorption du filtre en solution ou du produit appliqué sur un substrat visant à simuler le relief de la peau. L'efficacité contre les rayons UVB, UVA ou les deux, ou leurs effets sur la réponse cutanée, sont ensuite déterminés par le calcul, prenant en compte ou non le spectre d'action des radiations UV pour le dommage considéré.

**Exemple 25-1 : Evaluation du facteur de protection solaire (FPS) in vitro dans un solvant chimique**

La méthode de Sayre/ Agin et Diffey/ Robson, pratiquée depuis les années 1990, préconise une mesure comparative, à l'aide d'un spectroradiomètre à sphère intégratrice, de la transition de 290 nm à 400 nm par bandes de 5 nm, l'échantillon étant soumis au rayonnement UV d'une source stable et connue couvrant la totalité du spectre UV ( xénon non filtré).

Diffey et Robson pour évaluer par le calcul est la réponse érythèmale. La formule est la suivante :

$$FPS = \frac{\left(\sum_{290}^{400} E(\lambda) * \varepsilon\right)}{\left(\sum_{290}^{400} \frac{E(\lambda) * \varepsilon}{FPM(\lambda)}\right)}$$

$E(\lambda)$= Irradiation spectrale en $W.m^{-2}.nm^{-1}$ à 40°N soleil au zénith angle 20° $\varepsilon$= Capacité érythémateuse

$$\overline{FPM(\lambda)} = \frac{\left(\sum_{i=1}^{N} FPM(\lambda)i\right)}{N(\lambda)}$$

$N(\lambda)$= nombre de valeurs pour une longueur d'onde déterminée

La formule de Diffey et Robson a permis de déterminer le FPS à partir de la mesure de la transmittance entre 290 nm et 400 nm. La transmittance est mesurée en solution dans de l'acétate d'éthyle à une concentration de $10\mu g.ml^{-1}$ à l'aide d'un spectrophotomètre UV-visible (Varian CARY 50 Scan).

$$FPM(\lambda) = \frac{1}{T(\lambda)}$$

$T(\lambda)$= la transmitance à une longueur d'onde $\lambda$

Les résultats des mesures effectuées sont rassemblés dans le tableau 4.

**TABLEAU 4**

| Mesure du FPS in vitro dans un solvant chimique | |
|---|---|
| **Molécules** | **FPS** |
| **Parsol MCX®** | 20,78 |
| **Parsol 1789®** | 51,1 |
| **Tinosorb M®** | 54,07 |
| **Tinosorb S®** | 76,19 |
| **Composés de formule (I)** | |
| **JR65** | 68,21 |
| **JR77** | 47,16 |
| **JR113** | 67,74 |
| **JR173** | 69,02 |

**Exemple 25-2 : Evaluation du facteur de protection solaire (FPS) *in vitro* dans une formule primaire pour des mélanges comprenant des composés de formule (Ia) et des produits de référence**

Nous avons utilisé des plaques de PMMA (polymethylmethacrylate) (50x50 mm, fournisseur : Europlast)

La dose appliquée dans les protocoles d'évaluation des produits solaires in vivo ($2mg/cm^2$) est trop élevée pour le type de substrat employé ici. Une étude a monté que les meilleures corrélations avec le FPS étaient obtenues par application de 1,2 $mg/cm^2$.

Des gels ont été formulés à partir des triazines objets de la présente invention et de certains filtres de référence. Les molécules sont solubilisées dans le Transcutol® CG au bain-marie. La solution est ensuite placée sous agitation magnétique et un gélifiant, le Klucel HF (hydroxypropyl cellulose), est ajouté à la concentration de 2%. L'agitation est maintenue pendant 30 minutes. La mesure du FPS est effectuée à l'aide du spectrophotomètre Labsphère UV-1000 S Transmittance Analyser (Labsphère, North Sutton, USA).

Les résultats des mesures effectuées sont rassemblés dans le tableau 5.

**TABLEAU 5**

| **Mesure du FPS in vitro dans une formule primaire** | |
|---|---|
| **Mélange** | **FPS** |
| Parsol MCX®5% | 17,2 |
| JR65 3% | 10,9 |
| Tinosorb S® 3% | 9,8 |
| JR65 3%+ Tinosorb S® 3% | 19,4 |
| Parsol MCX®5%+ JR65 1% | 19,4 |
| Parsol MCX®5%+ JR67 1% | 27,9 |
| Parsol MCX®5%+ JR68 1% | 24,1 |
| Parsol MCX®5%+ JR77 1 % | 21,9 |
| Parsol MCX®5%+ JR113 1 % | 18,3 |
| JR65 3% | 9,8 |

**Exemple 26 : Etude de la photostabilité en solution dans un solvant chimique**

Nous avons utilisé un Suntest CPS+ (ATLAS, Linsengenicht/Altenhasslan, Germany). Le Suntest permet de reproduire le spectre solaire et donc de réaliser des expositions en section intérieure en temps voulu et sans contraintes météorologiques.

Réglage de la DEM (Dose Erythémale Minimale) :

L'irradiance du simulateur solaire a été soigneusement mesurée avec un spectroradiomètre (MSS 2044, Bielefeld, Germany). Les intensités des UVB et des UVA étaient respectivement de 0.49 mW/cm$^2$ et 6.32 mW/cm$^2$. La valeur de la DEM définie par le COLIPA est de 5,6 J/cm$^2$ en UV totaux (22). Les UV totaux (UVA+UVB) représentent 14,8% de l'énergie délivrée par la lampe (puissance 460W/m$^2$). Une dose d'irradiation équivalente à 1 DEM correspond à 37,83 J/cm$^2$ (en spectre total) délivrés par la lampe.

La durée d'essai avec le Suntest se calcule grâce à la formule suivante :

$$t = H / E$$

avec

E : Eclairement énergétique en W/m$^2$
H : Dose d'irradiation en J/m$^2$
t : Durée de l'essai en s.

Le réglage de la DEM sur le Suntest et la correspondance avec l'ensoleillement de 3 stations balnéaires sont indiqués au Tableau 6.

**TABLEAU 6**

| Ensoleillement | Extrême | Intense | Moyen |
|---|---|---|---|
| Lieu (21 juin) | Agadir | Toulon | La Baule |
| Nombre DEM/j | 20 | 10 | 5 |
| Dose d'irradiation correspondante sur le Suntest (en J/m$^2$) | 7566000 | 3783000 | 1891500 |
| Durée de l'essai | 4H34 | 2H17 | 1H08 |

**Mode opératoire:**

Les solutions de composés sont préparées en une concentration de 500 µg/mL dans le méthanol. 50 µL (soit 25 µg) de chacune des solutions sont déposés dans des cristallisoirs, puis irradiés dans le Suntest à 5, 10 et/ou 20DEM. Un témoin non irradié est préparé (dépôt de 50 µL de solution et ajout de 2,450 mL de méthanol). Le solvant s'évaporant lors de l'irradiation, les produits sont repris dans 2,5 ml de méthanol. Après l'irradiation, l'absorbance de chaque solution est mesurée au spectrophotomètre UV-visible (Varian CARY 50 Scan).

Les résultats de mesure de la photostabilité des composés de formule (I) sont regroupés dans le tableau 7.

**TABLEAU 7**

| | Photostabilité à 10DEM | | | Photostabilité à 20 DEM | | |
|---|---|---|---|---|---|---|
| **Molécule** | **moyenne** | **écart-type** | **écart type relatif** | **moyenne** | **écart type** | **écart type relatif** |
| Parsol 1789® | **57,04** | 6,62 | 11,60% | **6,26** | 3,52 | 56,26% |
| Parsol MCX® | **33,38** | 0,51 | 1.52% | **11,63** | 3,73 | 32,06% |
| JR89 | **97,39** | 2,25 | 2,31% | **98,72** | 1,44 | 1,46% |
| JR115 | **57,63** | 1,04 | 1,81% | **46,42** | 6,39 | 13,76% |
| JR18 | **82,86** | 0,68 | 0,82% | **57,37** | 9,26 | 16,14% |
| JR63 | **62,66** | 2,59 | 4,14% | **39,67** | 3,51 | 8,85% |
| JR65 | **96,91** | 2,26 | 2,33% | **85,5** | 5,81 | 6,80% |
| JR68 | **98,2** | 0,75 | 0,77% | **95,33** | 5,69 | 5,96% |
| JR70 | **44,51** | 2,78 | 6,25% | **30,83** | 5,35 | 17,34% |
| JR77 | **73,03** | 1,72 | 2,36% | **61,14** | 10,52 | 17,20% |
| JR107 | **58,45** | 0,22 | 0,37% | **47,81** | 15,76 | 32,95% |
| JR113 | **98,07** | 1 | 1,02% | **97,67** | 4,04 | 4,14% |
| JR114 | **92,07** | 0,9 | 0,98% | **91,43** | 0,38 | 0,41% |
| * comparatif | | | | | | |

**Exemple 27 : Etude de solubilité**

Les résultats de solubilité dans des solvants ou excipients utilisés en cosmétique sont regroupés dans le tableau 8.

**TABLEAU 8**

| Composés de formule (I) | | |
|---|---|---|
| **Molécules** | **Excipients** | **Solubilité (%)** |
| JR65 | **Myritol 318®** | 0,5 |
| | **Finsol V NT®** | 1 |
| | **Adipate d'isopropyle** | 2 |
| | **Butylèneglycol** | 5 |
| | **Hexylèneglycol** | 5 |
| | **Myristate d'isopropyle** | 5 |
| | **Cétiol V®** | 2 |
| | **Cétiol SN®** | 1 |
| | **Arlasolve DMI®** | 8 |
| | **PEG400®** | 5 |
| JR173 | **Finsol V NT®** | 7 |

(suite)

| Composés de formule (I) | | |
|---|---|---|
| **Molécules** | **Excipients** | **Solubilité (%)** |
| | **PEG400®** | 2 |
| **Arlasolve DMI®** = Diméthyl Isosorbide<br>**Cétiol SN®**= Cétéaryl Isononanoate<br>**Cétiol V®** = Décyl Oléate<br>**Finsol V NT®**= C12-C15 alkyl benzoate<br>**Myritol 318®**= Caprilic/ Capric Triglycérides<br>**PEG400®** = Polyéthylèneglycol ( n=400) | | |

**Exemple 28 : Exemple de formulation**

| Composition (émulsion H/E) | Quantité (g) |
|---|---|
| Sulfate de magnésium hydraté | 0,7 |
| Para méthoxy cinnamate d'éthyl hexyl | 5 |
| JR65 | 8 |
| Benzoate d'alcool en C12/C15 | 10 |
| Oxyde de titane | 3 |
| Triéthanolamine | Qs pH : 7 |
| Glycéride | 3 |
| Conservateurs | Qs |
| Eau déminéralisée | qsp 100 |

## Revendications

**1.** Utilisation de composés 5,6-diphényl-1,2,4-triaziniques de formule générale (I) :

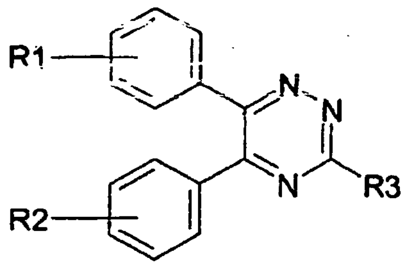

(I)

dans laquelle :

les liaisons pénétrantes dans le cycle indiquent une position de substitution indifférente en ortho, méta ou para, $R_1$ et $R_2$, identiques ou différents, représentent un atome d'hydrogène, de fluor, de chlore ou de brome, un groupe alkyle linéaire ou ramifié en $C_1$ à $C_{12}$, hydroxy, alcoxy linéaire ou ramifié en $C_1$ à $C_{18}$, poly(éthoxy)-alcoxy avec un fragment alkyle en $C_1$ à $C_4$ et le nombre de motif éthoxy compris entre 1 et 4, amino, ou mono- ou di-alkylamino avec un fragment alkyle en $C_1$ à $C_4$,

$R_3$ représente un atome de chlore, un groupe amino, phényle éventuellement substitué 1 à 3 fois par un radical hydroxy situé au moins en position para ou phényle éventuellement substitué 1 à 3 fois en position ortho, méta ou para, par un alcoxy en $C_1$ à $C_{12}$ ou un cyano et un groupe alkylimino avec un fragment alkyle en $C_1$ à $C_7$, comme filtres solaires actifs dans l'UV-A et/ou l'UV-B et/ou l'UV-C pour la peau humaine et/ou les cheveux.

**2.** Utilisation des composés tels que définis à la revendication 1, comme agents protecteurs de la lumière actifs dans l'UV-A et/ou l'UV-B et/ou l'UV-C, utiles dans l'industrie des matériaux synthétiques.

**3.** Utilisation selon la revendication 2, comme agents protecteurs de la lumière entrant dans la composition de matières plastiques, de verre ou de matières textiles.

**4.** Composés 5,6-diphényl-1,2,4-triaziniques de formule générale (Ia) :

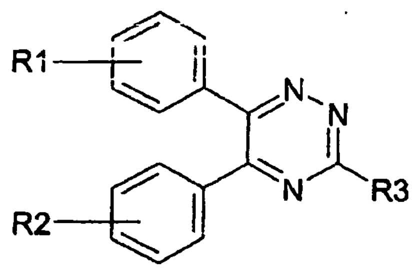

(Ia)

dans laquelle :

$R_1$ et $R_2$ représente un groupe $CH_3O$-, et
$R_3$ représente :

- un phényle substitué une ou plusieurs fois par un radical hydroxy situé au moins en position para,
- un phényle substitué en position ortho, méta ou para, par un radical choisi parmi un alcoxy en $C_1$ ou $C_{12}$ ou un cyano,
- ou un groupe alkylimino avec un fragment alkyle en $C_1$ à $C_7$.

**5.** Composés 5,6-diphényl-1,2,4-triaziniques selon la revendication 4 de formule (Ia), dans laquelle :

$R_1$ et $R_2$ représentent un groupe $CH_3O$- situé en position para, et
$R_3$ représente un groupe phényle substitué par un radical hydroxy en position para, ou par un cyano ou un alcoxy en $C_1$ à $C_{12}$ en position ortho, méta ou para.

**6.** Composés 5,6-diphényl-1,2,4-triaziniques selon les revendications 4 ou 5, choisis parmi :

JR65 : 3-(4-hydroxyphényl)-5,6-bis(4-méthoxyphényl)-1,2,4-triazine
JR70 : 3-(4-méthoxyphényl)-5,6-bis(4-méthoxyphényl)-1,2,4-triazine
JR89 : 3-(2-hydroxyphényl)-5,6-bis(4-méthoxyphényl)-1,2,4-triazine
JR107 : 3-(4-cyanophényl)-5,6-bis(4-méthoxyphényl)-1,2,4-triazine
JR113 : 3-(3-cyanophényl)-5,6-bis(4-méthoxyphényl)-1,2,4-triazine
JR114: 3-(2-cyanophényl)-5,6-bis(4-méthoxyphényl)-1,2,4-triazine
JR115: 3-(3,4,5-hydroxyphényl)-5,6-bis(4-méthoxyphényl)-1,2,4-triazine
JR117: [5,6-bis-(4-méthoxyphényl)-[1,2,4]-triazin-3-yl]heptylidine-amine
JR144 : 3-(3-cyanophényl)-5,6-diphényl-1,2,4-triazine
JR145 : 3-(2-cyanophényl) -5,6-diphényl-1,2,4-triazine
JR173 : 3-(4-dodécyloxyphényl)-5,6-bis(4-méthoxyphényl)-1,2,4-triazine.

**7.** Composition cosmétique anti-solaire active dans l'UV-A et/ou l'UV-B et/ou l'UV-C contenant une quantité efficace d'au moins un des composés 5,6-diphényl-1,2,4-triaziniques selon les revendications 4 ou 5 en association avec un excipient cosmétiquement acceptable, comprise de préférence entre de 0,1 % et 20 % en poids par rapport au poids total de la composition.

**8.** Composition cosmétique anti-solaire selon la revendication 7, **caractérisée en ce qu'**elle contient en outre un ou plusieurs autres filtres solaires complémentaires actifs dans l'UV-A et/ou dans l'UV-B et/ou dans l'UV-C.

## Claims

**1.** The use of 5,6-diphenyl-1,2,4-triazinic compounds of general formula (I):

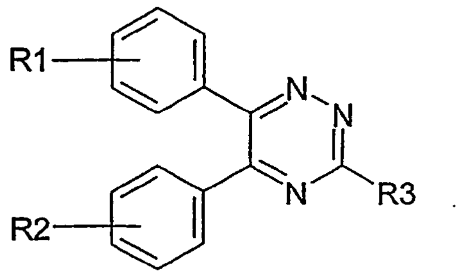

(I)

wherein:

the bonds that penetrate into the ring indicate an indifferent substitution position in ortho, meta or para,
$R_1$ and $R_2$, identical or different, represent a hydrogen, fluorine, chlorine or bromine atom, a $C_1$ to $C_{12}$ linear or branched alkyl group, a hydroxy group, a $C_1$ to $C_{18}$ linear or branched alkoxy group, a poly(ethoxy)alkoxy group with a $C_1$ to $C_4$ alkyl fragment and an ethoxy number ranging from 1 to 4, an amino group, or a mono- or di-alkylamino group with a $C_1$ to $C_4$ alkyl fragment,
$R_3$ represents a chlorine atom, an amino group, a phenyl group optionally substituted 1 to 3 times with a hydroxy radical located at least in para position, or a phenyl group optionally substituted 1 to 3 times in ortho, meta or para position with a $C_1$ to $C_{12}$ alkoxy or a cyano and an alkylimino group with a $C_1$ to $C_7$ alkyl fragment,
as sun filters active in UV-A and/or UV-B and/or UV-C for human skin and/or hair.

**2.** The use of compounds such as defined in claim 1 as light-protective agents active in UV-A and/or UV-B and/or UV-C, useful in the synthetic material industry.

**3.** The use according to claim 2 as light-protective agents incorporated into the composition of plastics, glass or textiles.

**4.** 5,6-Diphenyl-1,2,4-triazinic compounds of general formula (Ia) :

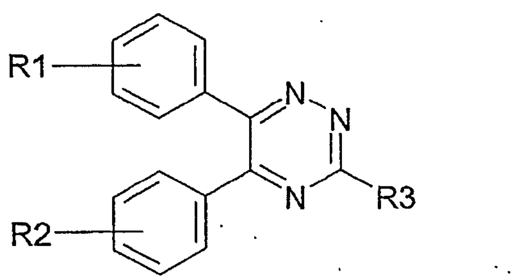

(Ia)

wherein:

$R_1$ and $R_2$ represent a $CH_3O$- group, and
$R_3$ represents:

- a phenyl group substituted one or several times by a hydroxy radical located at least in para position,
- a phenyl group substituted in ortho, meta or para position by a radical chosen among $C_1$ or $C_{12}$ alkoxy or a cyano,

- or an alkylimino group with $C_1$ to $C_7$ alkyl fragment.

**5.** 5,6-Diphenyl-1,2,4-triazinic compounds according to claim 4 of general formula (Ia), wherein:

$R_1$ and $R_2$ represent a $CH_3O$- group located in para position, and
$R_3$ represents a phenyl group substituted with a hydroxy radical in para position, or by a cyano or $C_1$ to $C_{12}$ alkoxy in ortho, meta or para position.

**6.** 5,6-Diphenyl-1,2,4-triazinic compounds according to claims 4 or 5, chosen among:

JR65: 3-(4-hydroxyphenyl)-5,6-bis(4-methoxyphenyl)-1,2,4-triazine
JR70: 3-(4-methoxyphenyl)-5,6-bis(4-methoxyphenyl)-1,2,4-triazine
JR89: 3-(2-hydroxyphenyl)-5,6-bis(4-methoxyphenyl)-1,2,4-triazine
JR107: 3-(4-cyanophenyl)-5,6-bis(4-methoxyphenyl)-1,2,4-triazine
JR113: 3-(3-cyanophenyl)-5,6-bis(4-methoxyphenyl)-1,2,4-triazine
JR114: 3-(2-cyanophenyl)-5,6-bis(4-methoxyphenyl)-1,2,4-triazine
JR115: 3-(3,4,5-hydroxyphenyl)-5,6-bis(4-methoxyphenyl)-1,2,4-triazine
JR117: [5,6-bis(4-methoxyphenyl)-[1,2,4]-triazin-3-yl]heptylidine-amine
JR144: 3-(3-cyanophenyl)-5,6-diphenyl-1,2,4-triazine
JR145: 3-(2-cyanophenyl)-5,6-diphenyl-1,2,4-triazine
JR173: 3-(4-dodecyloxyphenyl)-5,6-bis(4-methoxyphenyl)-1,2,4-triazine.

**7.** A cosmetic sunscreen composition active in UV-A and/or UV-B and/or UV-C containing an effective quantity of at least one 5,6-diphenyl-1,2,4-triazinic compound according to claims 4 or 5 in combination with a cosmetically acceptable excipient, preferably comprised between 0.1% and 20% by weight with respect to the total weight of the composition.

**8.** A cosmetic sunscreen composition according to claim 7, **characterized in that** it further contains one or more other complementary sun filters active in UV-A and/or UV-B and/or UV-C.

**Patentansprüche**

**1.** Verwendung von 5,6-Diphenyl-1,2,4-triazinVerbindungen der allgemeinen Formel (I):

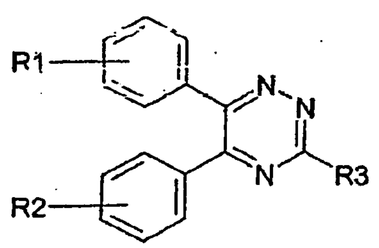

(I)

in welcher

die in den Ring eindringenden Bindungen eine indifferente Ortho-, Meta- oder Para-Substitutionsposition anzeigen,
$R_1$ und $R_2$, identisch oder unterschiedlich, ein, Wasserstoff-, Fluor-, Chlor- oder Bromatom, eine lineare oder verzweigte $C_1$-$C_{12}$-Alkyl-, Hydroxy-, lineare oder verzeigte $C_1$-$C_{18}$-Alcoxy-, Poly(ethoxy)-alcoxygruppe mit einem $C_1$-$C_4$-Alkylfragment und der Ethoxy-Motivanzahl zwischen 1 und 4 inklusive, Amino, oder Mono- oder Dialkylamino mit einem $C_1$-$C_4$-Alkylfragment darstellen,
R3 ein Chloratom, eine Amino-, Phenyl, eventuell 1 bis 3 Mal durch ein Hydroxyradikal substituiert, das sich mindestens an der Paraposition befindet, oder phenylgruppe eventuell 1 bis 3 Mal an der Ortho-, Meta-oder Paraposition, durch ein $C_1$-$C_{12}$-Alcoxy oder ein Cyano substituiert und eine Alkyliminogruppe mit einem $C_1$-$C_7$-Alkylfragment darstellt
als im UVA- und/oder UVB- und/oder UVC-Bereich aktive Sonnenfilter für die menschliche Haut und/oder die

Haare.

**2.** Verwendung von Verbindungen wie in Anspruch 1 definiert als im UVA - und/oder UVB - und/oder UVC Bereich aktive Lichtschutzmittel nützliche in der Industrie der Synthetischen Materialen.

**3.** Verwendung nach Anspruch 2 als Lichtschutzmittel vor in die Zusammensetzung von Kunststoffen, Glas oder Textilien eindringendem.

**4.** 5,6-Diphenyl-1,2,4-triazin-Verbindungen der allgemeinen Formel (Ia) :

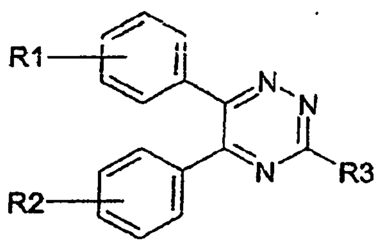

(Ia)

in welcher:

$R_1$ und $R_2$ eine $CH_3O$-Gruppe darstellen,
$R_3$ darstellt:

- ein Phenyl, das einmal oder mehrere Male von einem Hydroxyradikal substituiert wurde, das sich mindestens an der Paraposition befindet,
- ein Phenyl, das an der Ortho-, Meta- oder Paraposition von einem Radikal substituiert wurde, das aus einem $C_1$-$C_{12}$-Alcoxy oder einem Cyano ausgewählt wurde,
- oder eine Alkyliminogruppe mit einem $C_1$-$C_7$-Alkylfragment.

**5.** 5,6-Diphenyl-1,2,4-trianzin-Verbindungen nach Anspruch 4 der Formel (Ia), in welcher:

$R_1$ und $R_2$ eine an der Paraposition befindliche $CH_3O$-Gruppe darstellen, und
$R_3$ eine Phenylgruppe darstellt, die an der Paraposition von einem Hydroxyradikal, oder von einem Cyano oder einem $C_1$-$C_{12}$-Alcoxy an der Ortho-, Meta- oder Paraposition substituiert wurde.

**6.** 5,6-Diphenyl-1,2,4-triazin-Verbindungen nach den Ansprüchen 4 oder 5, ausgewählt aus:

JR65: 3-(4-Hydroxyphenyl)-5,6-bis(4-methoxyphenyl)-1,2,4-triazin,
JR70: 3-(4-Methoxyphenyl)-5,6-bis(4-methoxyphenyl)-1,2,4-triazin,
JR89: 3-(2-Hydroxyphenyl)-5,6-bis(4-methoxyphenyl)-1,2,4-triazin,
JR107: 3-(4-Cyanophenyl)-5,6-bis(4-methoxyphenyl)-1,2,4-triazin,
JR113: 3-(3-Cyanophenyl)-5,6-bis(4-methoxyphenyl)-1,2,4-triazin,
JR114: 3-(2-Cyanophenyl)-5,6-bis(4-methoxyphenyl)-1,2,4-triazin,
JR115: 3-(3,4,5-Hydroxyphenyl)-5,6-bis(4-methoxyphenyl)-1,2,4-triazin,
JR117: [5,6-bis-(4-Methoxyphenyl)-[1,2,4]-triazin-3-yl]heptylidin-amin,
JR144: 3-(2-Cyanophenyl)-5,6-diphenyl-1,2,4-triazin,
JR145: 3-(2-Cyanophenyl)-5,6-diphenyl-1,2,4-triazin,
JR173: 3-(4-Dodecyloxyphenyl)-5,6-bis(4-methoxyphenyl)-1,2,4-triazin.

**7.** Kosmetische, im WA- und/oder UVB- und/oder UVC-Bereich aktive Sonnenschutz-Zusammensetzung, die eine wirksame Menge von mindestens einer der 5,6-Diphenyl-1,2,4-triazin-Verbindungen nach den Ansprüchen 4 oder 5 enthält in Kombination mit einem kosmetisch akzeptablen Träger, vorzugsweise zwischen 0,1 und 20 Gew.-% im Verhältnis zum Gesamtgewicht der Zusammensetzung inklusive.

**8.** Kosmetische Sonnenschutz-Zusammensetzung nach Anspruch 7, **dadurch gekennzeichnet, dass** sie weiterhin einen oder mehrere andere ergänzende im UVA- und/oder UVB- und/oder UVC-Bereich aktive Sonnenfilter enthält.

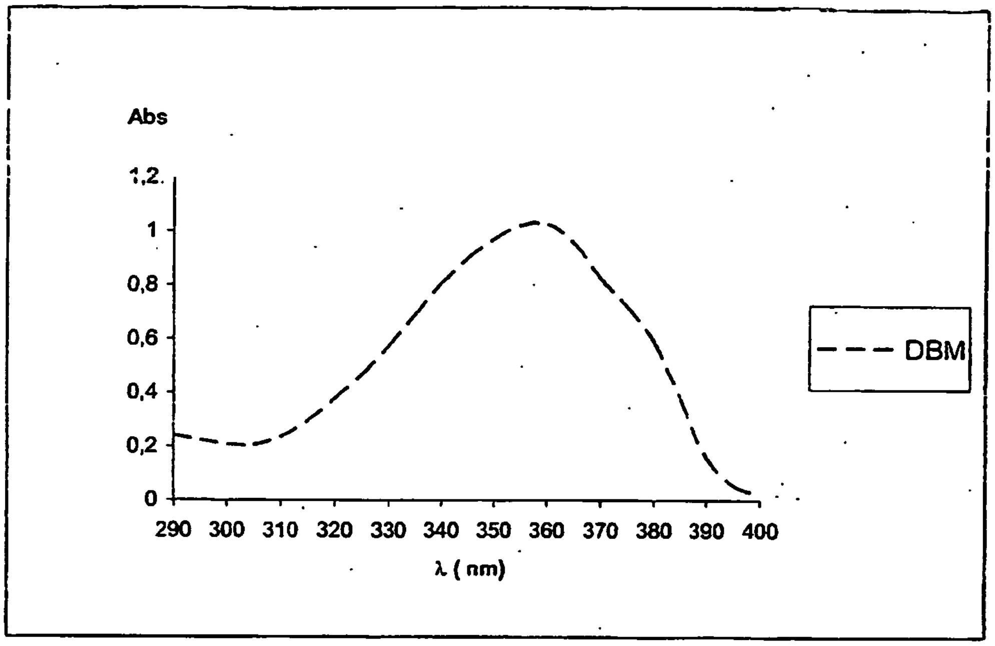


FIGURE 1

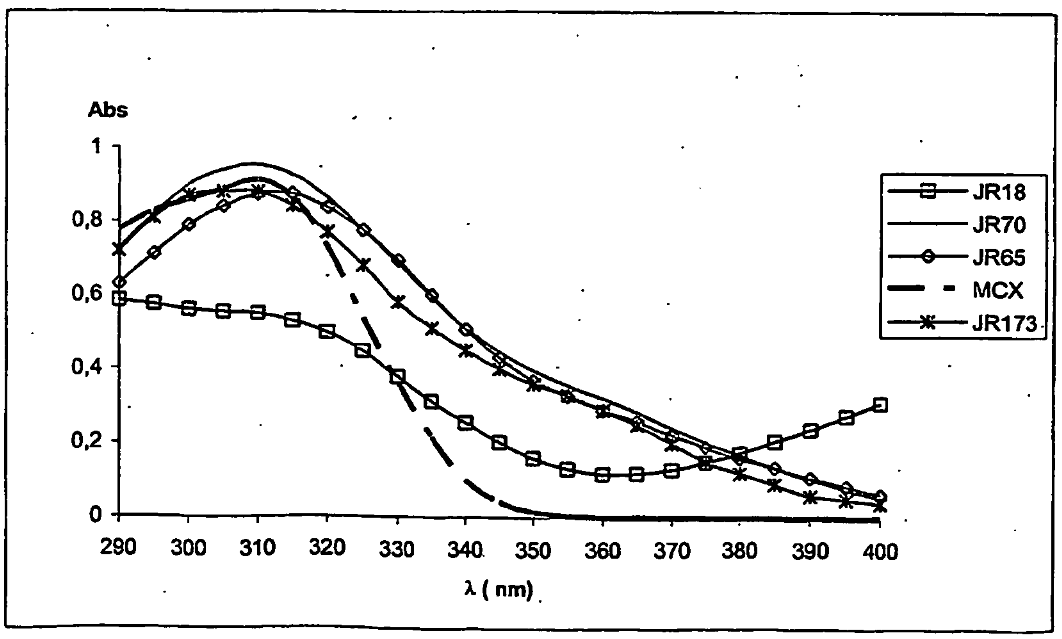


FIGURE 2

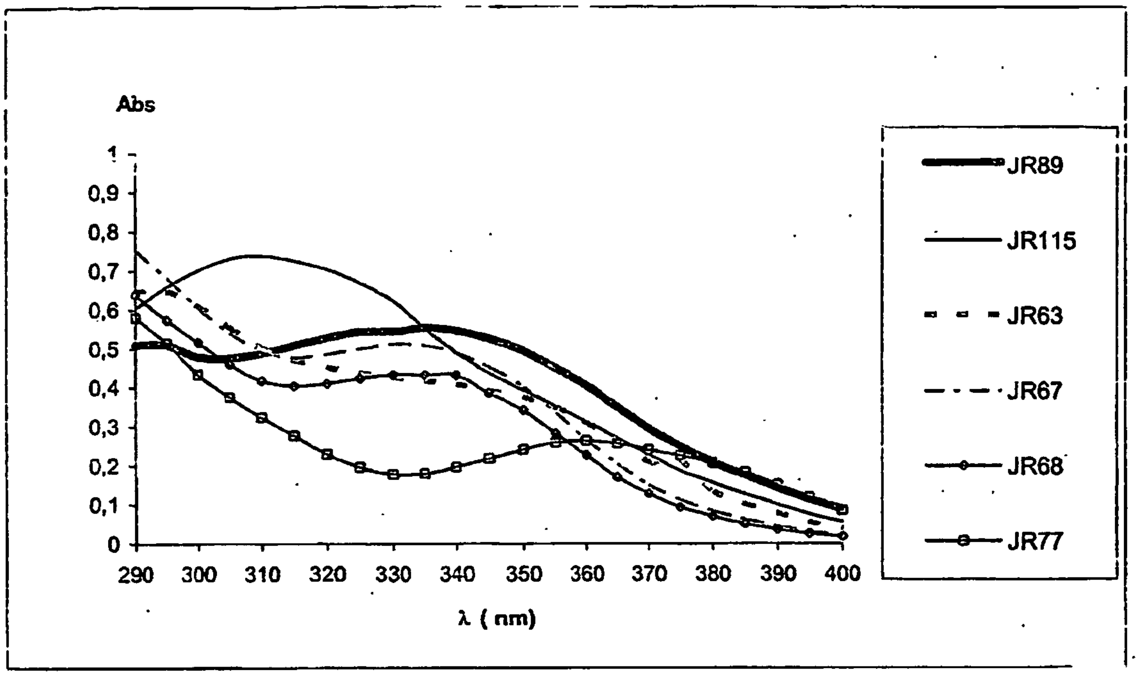


FIGURE 3

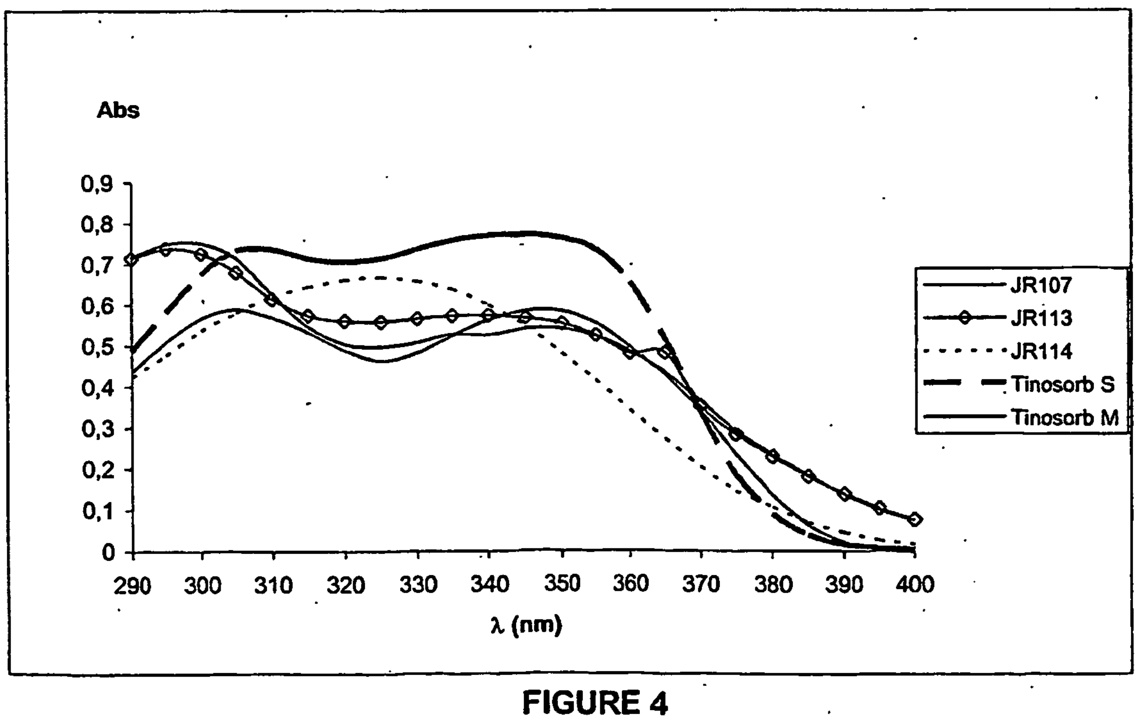


FIGURE 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2803194
- US 3948894 A


### Littérature non-brevet citée dans la description


- **G. Pitet ; H. Cousse ; G. Mouzin.** *Boll. Chim. Farm.,* 1980, vol. 119, 469
- **C. Tuzin ; M. Ogliaruso ; E. I. Becker.** *Org. Syn.,* 1961, vol. 41, 3
- **E. C. Taylor ; L. G. French.** *J. Org. Chem,* 1989, vol. 54, 1245